# EUROPÄISCHE PATENTSCHRIFT

(11) **EP 0 867 193 B1**
(45) Veröffentlichungstag und Bekanntmachung des Hinweises auf die Patenterteilung: **18.12.2002**
(21) Anmeldenummer: 98105386.1
(22) Anmeldetag: 25.03.1998
(51) Int. Cl.: A61L 31/00

(54) **Folie für die Medizintechnik**
Foil for medical use
Feuille à usage médical

(30) Priorität: 27.03.1997 DE 19713011
(43) Veröffentlichungstag der Anmeldung: 30.09.1998
(73) Patentinhaber: FRIADENT GmbH, 68229 Mannheim (DE)
(72) Erfinder: Haessler, Dieter Dr., 55276 Oppenheim (DE)
(74) Vertreter: Schmitt, Meinrad, Dipl.-Ing.

(56) Entgegenhaltungen:
- EP-A- 0 520 177
- EP-A- 0 621 018
- EP-A- 0 809 979
- DE-A- 19 721 192
- DE-C- 4 313 192
- FR-A- 2 610 512
- FR-A- 2 713 090
- US-A- 5 348 788

## Beschreibung

Die Erfindung bezieht sich auf eine Folie für die Medizintechnik gemäß den im Oberbegriff des Patentanspruchs 1 angegebenen Merkmalen.

Aus der FR-A- 2 713 090 ist eine derartige Folie oder Membran zur gesteuerten Geweberegeneration bekannt, welche aus Metall besteht, und zwar insbesondere aus reinem Titan. Diese Folie hat jedoch eine vergleichsweise große Federelastizität und ist derart vorgeformt, daß die dem zu regenerierenden Knochengewebe zugewandte Seite konkav ausgebildet ist. Die durch die Federelastizität bedingte Rückstellbarkeit erschwert die Anpassung und Plazierung der Folie an bzw. auf dem zu regenerierenden Knochengewebe. Die vorgeformte gebogene Folie weist an ihren Längsrändern Verbindungskörper aus biokompatiblem Material auf, wie insbesondere Silikon auf, um eine Abdichtung und / oder Stabilisation der Membran bezüglich des zu regenerierenden Gewebes zu erzielen. Je nach Ausbildung des Knochengewebes müssen derart gebogene Titanfolien mit unterschiedlichen Krümmungsradien bereitgestellt werden, damit eine dichte und / oder federnde Anlage der Folie am Knochengewebe erzielt werden kann. Eine Verformung der Folie zwecks Anpassung an vorhandene Knochenstrukturen ist nicht ohne weiteres möglich. Zudem muß ein vergleichsweise großer Bereich des Knochens vom Bindegewebe oder der Haut freigelegt sein, damit die genannten Verbindungskörper in der erforderlichen Weise am Knochen zur Anlage gebracht werden können. Diese Folie gelangt daher vor allem bei Knochen der Gliedmaßen wie Arme und Beine zur Anwendung und ist nicht ohne weiteres für vergleichsweise kleine Knochendefekte, wie insbesondere in der Dentalmedizin, einsetzbar.

In der Medizintechnik gelangen derartige Folien oder Membranen zum Abdecken von Knochendefekten zum Einsatz, um das Einsprießen von Bindegewebe in den Defektraum zu verhindern. Auch wurden bisher Kunststoffmembranen vorgesehen, deren mechanische Stabilität den heutigen Anforderungen nicht mehr im vollen Umfang entspricht. Mit den bekannten Kunststoffmembranen kann bei Knochendefekten Augmentat fixiert werden, welches die knöcherne Regeneration des Defektes verbessern soll. In der Medizintechnik ist Titan aufgrund seiner Bio-Kompatibilität für Implantate hinlänglich bekannt. Titan und seine Legierungen weisen besonders günstige chemische und mechanische Eigenschaften auf, welche den Einsatz in der Medizintechnik ermöglichen.

Ferner ist aus der EP-A-0 621 018 eine Prothese mit einer Anzahl von übereinander angeordneten Titanfolien bekannt. Diese Titanfolien enthalten Durchbrechungen, in welche das umgebende Knochengewebe zur Fixierung der Prothese von der dem Knochen abgewandten Seite der Folien einwachsen kann. Eine Abdeckung oder Trennung des Knochens bezüglich des den Knochen umgebenden Epithel- oder Bindegewebes erfolgt hierbei nicht. Um das Einwachsen des Knochengewebes in die Durchbrechung der übereinander gestapelten Folien zu ermöglichen, weisen die Durchbrechungen vergleichbare große Abmessungen auf, so daß ggfs. Epithel- oder Bindegewebe hindurchwachsen könnte, was bei Abdeckfolien zu vermeiden ist.

Des weiteren ist aus der EP-A-0 809 979 eine Folie zur Abdeckung von Knochendefektstellen bekannt. Diese Folie ist durch thermische Behandlung oder chemische Abtragung der beim Walzen verdichteten Oberflächen unelastisch, spannungsfrei und bleibend verformbar ausgebildet. Die dem Knochen abgewandte Oberfläche der Folie ist aufgerauht oder geriffelt, um einen guten Halt für das umgebende Binde- oder Weichgewebe zu ermöglichen.

Der Erfindung liegt die Aufgabe zugrunde, der genannten Art dahingehend weiterzubilden, daß eine gute mechanische Stabilität erreicht und eine einfache und funktionsgerechte Handhabung ermöglicht wird. Die Folie soll gut verformbar und problemlos an die anatomischen Gegebenheiten anpaßbar sein. Die Folie soll ferner formstabil sein und eine ungestörte Osteogenese ermöglichen. Die Folie soll vor allem bei kleinen Knochendefekten, insbesondere in der Dentalmedizin zum Aufbau und zur Regeneration des Kieferknochens verwendbar sein.

Die Lösung dieser Aufgabe erfolgt gemäß den Merkmalen des Patentanspruchs 1.

Die erfindungsgemäße Folie besteht aus Titan oder einer Titanlegierung und ist plastisch verformbar, so daß eine problemlose Anpassung an die anatomischen Gegebenheiten gewährleistet ist. Die Folie besitzt eine Vorprägung oder Strukturierung, die als in Winkeln zueinander angeordnete Linienstrukturen umgeben ist und eine bleibende plastische Verformbarkeit der Folie gewährleisten. Die Linienstrukturen weisen Höhendifferenzen im Bereich zwischen 100 bis 500µm oder zwischen 200 bis 800µm auf, wodurch eine hohe Formstabilität und gleichwohl eine bleibende plastische Verformbarkeit gewährleistet ist.

Ferner weist die Folie eine Dicke im Bereich zwischen 10µm und 100µm auf. Die bleibende plastische Verformbarkeit kann durch Wärmebehandlung und der damit verbundenen Reduzierung der Federelastizität des Titans oder der Titanlegierung verbessert werden.

Durch Verformen und Beschneiden der Titanfolie ist diese leicht den anatomischen Gegebenheiten anpaßbar, und sie behält die gewählte Form auch nach der Plazierung über einen Knochendefekt. Aufgrund der im Vergleich mit bekannten Kunststoffmembranen wesentlich höheren Stabilität werden Defekte, insbesondere Risse oder Löcher, beim Anpassen und Plazieren mit hoher Sicherheit vermieden. Selbst große Knochendefekte können mit der Titanfolie sicher und formstabil abgedeckt werden. Mit der formstabilen Titanfolie kann selbst bei zweiwandigen Knochendefekten, und zwar insbesondere in der Dentalmedizin, eine sichere Augmentat-Fixierung gewährleistet werden. Darüber hinaus schützt die erfindungsgemäße, bleibend verformbare Titanfolie in besonders zweckmäßiger Weise das sich im Defektraum organisierende Gewebe vor Krafteinflüssen und Membranstützen sind in der Regel nicht erforderlich.

Die Folie ist derart vorgeprägt, daß erhabene bzw. vertiefte Bereiche vorhanden sind. Es ist eine insbesondere rechteck- oder rautenförmige Linienstruktur vorgegeben, und zwar mit Längen vorzugsweise zwischen 0,2 bis 1 mm der Kanten der derart gebildeten Bereiche. Bezeichnet man die zwischen den rasterförmigen Linienstrukturen jeweils vorhandenen Bereiche als erhaben bezüglich der Linienstruktur, so ist eine Höhendifferenz vorgegeben, und zwar insbesondere zwischen 0,1 bis 0,5 mm oder zwischen 0,2 bis 0,8 mm. Ferner ist in einer zweckmäßigen Ausgestaltung die Folie mikroporös ausgebildet, wobei die Perforation insbesondere mittels eines Lasers erfolgt ist. Die Oberflächen der Folie weisen eine vorgegebene Rauhigkeit auf, und infolge der mikroporösen Ausbildung wird in optimaler Weise ein Nährstoffaustausch und / oder eine Fixierung des Bindegewebes gewährleistet. Die Außenkontur der Folie ist in besonders zweckmäßiger Weise abgerundet, sei es kreisförmig, oval oder elipsenförmig, so daß vor allem in der Dentalmedizin eine besonders vorteilhafte Anordnung im Bereich des Kieferknochens, insbesondere im Bereich des Knochenkamms, zur Regeneration von Knochengewebe für die Implantation eines Implantatkörpers in einer Zahnlücke vorgenommen werden kann.

Weiterbildungen und besondere Ausgestaltungen der Erfindung sind in den Unteransprüchen sowie der nachfolgenden Beschreibung eines besonderen Ausführungsbeispiels näher erläutert.

Die erfindungsgemäße Titanfolie zeichnet sich besonders durch eine geringe Rückstellung nach einer Biegung oder Deformation aus. Die Rückstellung ist zweckmäßig kleiner als 20% und vorzugsweise geringer als 10%.

Ein besonderes Ausführungsbeispiel der vorgeschlagenen Titanfolie wurde zur Biegeund Plastizitätsprüfung um einen Stab mit einem Durchmesser von 5 mm um 180° gebogen. Die Titanfolie besaß eine Dicke von 15µm, eine Länge von 30 mm und eine Breite von 10 mm, wobei die Breite in Richtung der Längsachse des Stabes gemessen wurde. Die Biegung über 180° wurde für 30 sec. fixiert. Danach ergab sich eine Rückstellung < als 45°.

Die erfindungsgemäße Titanfolie besitzt eine wesentlich geringere Rückstellung als eine herkömmliche Folie mit gleichen Abmessungen, bei welcher die Rückstellung > 90° gemessen wurde. Die erfindungsgemäße sowie die herkömmliche Folie wiesen im übrigen die gleichen Abmessungen und jeweils eine Materialdicke von 15 µm auf.

In einer besonderen Ausbildung der Erfindung ist die Oberfläche der Folie mit einer feinen Rauhung versehen, und zwar vorzugsweise durch feine Mikroporen. Die insbesondere punktuell vorgesehene Mikrostruktur wird durch Laserperforation erzeugt. Somit wird nicht nur die Rauhigkeit vorgegeben, sondern infolge der Porösität ein Nährstoffaustausch, Zellaustausch und letztendlich eine verbesserte Bindegewebfixierung gewährleistet. Die feine Rauhung der Oberfläche begünstigt und stabilisiert die Anlagerung des sich neu bildenden Bindegewebes. Aufgrund der punktuellen Mikrostruktur wird die federelastische Rückstellung weiter reduziert und die Plastifizierung verbessert.

Die Folie ist durch Linienstrukturen vorgeprägt, welche bevorzugt rechtwinkelig zueinander sind. In einer anderen erfindungsgemäßen Ausgetaltung ist eine Rautenstruktur vorgegeben. Unabhängig von der jeweiligen Ausbildung der Linienstrukturen ergeben diese eine verbesserte Plastifizierung der Folie und eine Minimierung der Federelastizität. Als zweckmäßig haben sich Abstände derartiger Strukturen, beispielsweise deren Maxima im Bereich zwischen 300 und 1000 µm, vorzugsweise zwischen 400 und 800 µm erwiesen. Die Höhendifferenz der Maxima und Minima derartiger Strukturen liegt erfindungsgemäß zwischen 200 und 800 µm, vorzugsweise zwischen 300 und 700 µm. In einer anderen Ausgestaltung der erfindungsgemäßen Folie ist die Höhendifferenz im Bereich zwischen 100 bis 500 µm vorgegeben. Die vorgegebene Linienstruktur gewährleistet verbesserte mechanische Eigenschaften, wobei ferner die bleibende plastische Verformbarkeit optimiert wird.

Des weiteren besteht die erfindungsgemäße Titanfolie aus Reintitan. Gegebenenfalls sind nur geringe Beimengungen von Kohlenstoff, maximal 0,1%, und Sauerstoff maximal 0,4%, vorzugsweise maximal 0,2% enthalten. Ferner sind die Beimengungen von Stickstoff kleiner als 0,05%, bevorzugt kleiner 0,03% und auch eventuelle Eisenbeimengungen betragen höchstens 0,5%, vorzugsweise höchstens 0,3 Gew.%. Ferner liegt die Zugfestigkeit der erfindungsgemäßen Folie im Bereich zwischen 200 und 500 MPa und vorzugsweise zwischen 250 und 450 MPa.

Des weiteren liegt die Streckgrenze der vorgeschlagenen Titanfolie im Bereich zwischen 200 und 420 MPa, vorzugsweise zwischen 220 und 300 MPa. Schließlich liegt die Dehnung im Bereich zwischen 20 und 35%, vorzugsweise zwischen 25 bis 30%.

Die erfindungsgemäße Folie ist in bevorzugter Weise nicht rechteckförmig ausgebildet, sondern weist eine gebogene Umfangskontur auf, insbesondere eine ovale, elipsenförmige oder kreisförmige Kontur. Damit ist vor allem zur Regeneration von flächenmäßig kleinen Knochendefekten, wie insbesondere in der Dentalmedizin bzw. der Kieferchirurgie, die Anpassung und die Anordnung selbst in eine Lücke eines einzelnen Zahnes problemlos durchführbar. Die Handhabung der Folie wird hierdurch nicht unerheblich verbessert. Des weiteren sind in besonders zweckmäßiger Weise im Bereich der Außenkontur radiale Einschnitte in der Folie vorgesehen, wodurch die Handhabung weiter verbessert wird. Die Tiefe der Einschnitte ist insbesondere im Bereich zwischen 0,2 bis 0,5 mm, vorzugsweise zwischen 0,3 bis 0,4 mm, vorgegeben. Die Abstände der Einschnitte über die Außenkontur bzw. den Umfang der Folie sind gleichfalls definiert vorgegeben, wobei Abstände im Bereich zwischen 5 bis 12 mm, insbesondere zwischen 7 bis 9 mm, sich als besonders zweckmäßig erwiesen haben.

## Patentansprüche

1. Folie für die Medizintechnik, welche zum Abdecken von Knochendefekten, insbesondere zur Regeneration und Augmentation von Knochen vorgesehen ist und aus Titan oder einer Titanlegierung besteht und verformbar ist,
**dadurch gekennzeichnet, daß** eine Vorprägung oder Strukturierung als in Winkeln zueinander angeordnete Linienstrukturen vorgegeben ist, welche orthogonal zur Folienoberfläche Höhendifferenzen im Bereich zwischen 100 bis 500 µm oder zwischen 200 bis 800µm aufweist, und daß die Dicke der Folie im Bereich zwischen 10µm bis 100µm liegt, wobei eine bleibende plastische Verformbarkeit gegeben ist.

2. Folie nach Anspruch 1, **dadurch gekennzeichnet, daß** die Linienstrukturen rechtwinklig zueinander angeordnet sind.

3. Folie nach Anspruch 1 oder 2, **dadurch gekennzeichnet, daß** sie aus Reintitan besteht.

4. Folie nach einem der Ansprüche 1 bis 3, **dadurch gekennzeichnet, daß** die Dicke der Folie im Bereich zwischen 10µm bis 20µm oder zwischen 20 bis 40µm liegt.

5. Folie nach einem der Ansprüche 1 bis 4, **dadurch gekennzeichnet, daß** bei Biegung der Folie die Rückstellung kleiner als 10% beträgt und daß durch Wärmebehandlung die genannte Rückstellung und die bleibende plastische Verformung vorgegeben ist.

6. Folie nach einem der Ansprüche 1 bis 5, **dadurch gekennzeichnet, daß** bei Biegung der Folie mit einem Biegeradius von 5mm über einen Winkelbereich von 180° die Rückstellung kleiner als 45° beträgt, wobei die Fixierung in der Biegestellung um 180° während einer Zeit von wenigstens 20 sec., zweckmäßig für 30 sec., erfolgt.

7. Folie nach einem der Ansprüche 1 bis 6, **dadurch gekennzeichnet, daß** die Oberfläche der Folie zumindest auf einer Seite, vorzugsweise auf der für das Bindegewebe vorgesehenen Seite, eine vorgegebene Rauhigkeit und/oder zumindest punktuell mikroporöse Struktur aufweist.

8. Folie nach einem der Ansprüche 1 bis 7, **dadurch gekennzeichnet, daß** die Abstände der Linienstrukturen im Bereich zischen 300 bis 1000µm vorgegeben sind.

9. Folie nach einem der Ansprüche 1 bis 7, **dadurch gekennzeichnet, daß** die Linienstrukturen im Bereich zwischen 200 bis 800µm vorgegeben sind.

10. Folie nach einem der Ansprüche 1 bis 9, **dadurch gekennzeichnet, daß** die Linienstrukturen orthogonal zur Folienoberfläche Höhendifferenzen im Bereich zwischen 300 und 700µm aufweisen

11. Folie nach einem der Ansprüche 1 bis 10, **dadurch gekennzeichnet, daß** die Außenkontur teilweise abgerundet ausgebildet ist.

12. Folie nach einem der Ansprüche 1 bis 10, **dadurch gekennzeichnet, daß** die Außenkontur abgerundet ausgebildet ist.

13. Folie nach einem der Ansprüche 1 bis 11, **dadurch gekennzeichnet, daß** an den Rändern Einschnitte vorgesehen sind.

## Claims

1. A foil for medical technology, which is provided for covering bone defects, in particular for the regeneration and augmentation of bones, and is made of titanium or a titanium alloy and is deformable,
**characterised in that** pre-embossing or structuring is carried out to produce linear structures arranged at angles to one another and having height differences in the range between 100 and 500 µm or between 200 and 800 µm orthogonal to the foil surface, and **in that** the thickness of the foil is in the range between 10 and 100 µm, a permanent plastic deformation being produced.

2. A foil according to Claim 1, **characterised in that** the linear structures are arranged at right angles to one another.

3. A foil according to Claim 1 or 2, **characterised in that** it is made of pure titanium.

4. A foil according to one of Claims 1 to 3, **characterised in that** the thickness of the foil is in the range between 10 µm and 20 µm or between 20 µm and 40 µm.

5. A foil according to one of Claims 1 to 4, **characterised in that**, upon bending the foil, the return is less than 10%, and **in that** the said return and the permanent plastic deformation are produced by heat treatment.

6. A foil according to one of Claims 1 to 5, **characterised in that**, upon bending the foil with a bending radius of 5 mm over an angular range of 180°, the return is less than 45°, the bent position through 180° being fixed over a time of at least 20 seconds, advantageously for 30 seconds.

7. A foil according to one of Claims 1 to 6, **characterised in that**, on at least one side, preferably the side provided for the connective tissue, the surface of the foil has a predetermined roughness and/or a structure which is microporous, at least at certain points.

8. A foil according to one of Claims 1 to 7, **characterised in that** the spacings between the linear structures are in the range between 300 and 1000 µm.

9. A foil according to one of Claims 1 to 7, **characterised in that** the linear structures are in the range between 200 and 800 µm.

10. A foil according to one of Claims 1 to 9, **characterised in that** the linear structures have height differences in the range between 300 and 700 µm orthogonal to the foil surface.

11. A foil according to one of Claims 1 to 10, **characterised in that** the outer contour is of a partially rounded construction.

12. A foil according to one of Claims 1 to 10, **characterised in that** the outer contour is of a rounded construction.

13. A foil according to one of Claims 1 to 11, **characterised in that** incisions are provided along the edges.

## Revendications

1. Feuille à usage médical, qui est prévue pour recouvrir des défauts osseux, en particulier pour la régénération et l'augmentation osseuses, et est composée de titane ou d'un alliage de titane et est déformable,
***caractérisée en ce qu****'un* pré-gaufrage ou une structuration est présent sous la forme de structures linéaires disposées selon des angles entre elles, qui présente perpendiculairement à la surface de la feuille des différences de hauteur comprises entre 100 et 500 µm ou entre 200 et 800 µm, et ***en ce que*** l'épaisseur de la feuille est comprise entre 10 µm et 100 µm, une déformabilité plastique résiduelle étant présente.

2. Feuille selon la Revendication 1, ***caractérisée en ce que*** les structures linéaires sont disposées perpendiculairement les unes aux autres.

3. Feuille selon la Revendication 1 ou 2, ***caractérisée en ce qu'***elle est composée de titane pur.

4. Feuille selon l'une quelconque des Revendications 1 à 3, ***caractérisée en ce que*** l'épaisseur de la feuille est comprise entre 10 µm et 20 µm, ou entre 20 µm et 40 µm.

5. Feuille selon l'une quelconque des Revendications 1 à 4, ***caractérisée en ce qu***'en cas de flexion de la feuille, la réaction est inférieure à 10 %, et ***en ce que*** ladite réaction et la déformation plastique résiduelle sont imposées par traitement thermique.

6. Feuille selon l'une quelconque des Revendications 1 à 5, ***caractérisée en ce qu***'en cas de flexion de la feuille avec un rayon de flexion de 5 mm sur un angle de 180°, la réaction est inférieure à 45°, la fixation dans la position à 180° s'effectuant pendant une durée d'au moins 20 secondes,et de manière préférée de 30 secondes.

7. Feuille selon l'une quelconque des Revendications 1 à 6, ***caractérisée en ce que*** la surface de la feuille présente au moins d'un côté, de préférence du côté prévu pour le tissu conjonctif, une rugosité prédéterminée et/ou une structure au moins ponctuellement microporeuse.

8. Feuille selon l'une quelconque des Revendications 1 à 7, ***caractérisée en ce que*** les distances entre les structures linéaires sont comprises entre 300 et 1000 µm.

9. Feuille selon l'une quelconque des Revendications 1 à 7, ***caractérisée en ce que*** les structures linéaires sont comprises entre 200 et 800 µm.

10. Feuille selon l'une quelconque des Revendications 1 à 9, ***caractérisée en ce que*** les structures linéaires présentent, perpendiculairement à la surface de la feuille, des différences de hauteur comprises entre 300 et 700 µm.

11. Feuille selon l'une quelconque des Revendications 1 à 10, ***caractérisée en ce que*** le contour extérieur est réalisé partiellement arrondi.

12. Feuille selon l'une quelconque des Revendications 1 à 10, ***caractérisée en ce que*** le contour extérieur est réalisé arrondi.

13. Feuille selon l'une quelconque des Revendications 1 à 11, ***caractérisée en ce que*** des découpes sont prévues sur les bords.
